# EUROPEAN PATENT APPLICATION

(11) **EP 3 275 989 A1**
(43) Date of publication of application: **31.01.2018**
(21) Application number: 16181325.8
(22) Date of filing: 26.07.2016
(51) Int. Cl.: C11D 3/386, C11D 11/00

(54) **AUTOMATIC DISHWASHING DETERGENT COMPOSITION**

(71) Applicant: The Procter & Gamble Company, Cincinnati, OH 45202 (US)
(72) Inventor: PEREZ-PRAT VINUESA, Eva Maria, Newcastle upon Tyne, NE12 9TS (GB); SOUTER, Philip Frank, Newcastle upon Tyne, NE12 9TS (GB)
(74) Representative: Yorquez Ramirez, Maria Isabel

(57) **Abstract**

A phosphate-free automatic dishwashing cleaning composition comprising a protease wherein the protease is a variant of a parent protease having the amino acid sequence of SEQ ID NO:1 and the variant protease has at least 90% identity with the amino acid sequence of SEQ ID NO:1 wherein the protease comprises at least one amino acid residue with a carboxylic acid side chain in the active site loop region from position 95 to 103 (BPN' numbering) with the proviso that the variant protease does not have 100% identity with SEQ ID NO:3, SEQ ID NO:4, or SEQ ID NO: 5.

## Description

### FIELD OF THE INVENTION

The present invention is in the field of detergents. In particular, it relates to a phosphate-free automatic dishwashing detergent composition comprising a protease. The composition provides improved cleaning of burnt-on sugary food soils even at low temperatures and short cycles.

### BACKGROND OF INVENTION

Some soils such as burnt-on sugary foods soils are very difficult to remove from dishware in automatic dishwashing. There is a permanent desire to improve the performance of automatic dishwashing compositions, their environmental profile and to reduce the energy required by the automatic dishwashing process.

The object of the present invention is to provide a phosphate-free automatic dishwashing composition that provides good cleaning of burnt-on sugary food soils even at low temperatures and short cycles.

### SUMMARY OF THE INVENTION

According to the first aspect of the present invention, there is provided a phosphate-free automatic dishwashing cleaning composition. The composition comprises a protease wherein the protease is a variant of a parent protease. The parent protease has the amino acid sequence of SEQ ID NO:1 (subtilisin GG36 from Bacillus lentus) and the variant protease has at least 90%, preferably at least 95% and more preferably at least 97% identity with the amino acid sequence of SEQ ID NO:1. The variant protease comprises at least one amino acid residue with a carboxylic acid side chain, i.e. aspartic acid and/or glutamic acid, in the active site loop region from position 95 to 103 (BPN' numbering). The variant protease does not have 100% identity with SEQ ID NO:3, SEQ ID NO:4, or SEQ ID NO: 5. The composition provides improved removal of burnt-on sugary food soils, such as crème brûlée even at low temperatures and short cycles. The composition is preferably free of anionic surfactant and preferably has a pH above 9.5, preferably from about 10.5 to about 12, as measured in 1% by weight aqueous solution at 25°C. This high pH contributes to the removal of burnt-on sugary soils.

According to the second, third and fourth aspects of the invention there are provided method of automatic dishwashing using the composition of the invention for the removal of burnt-on sugary soils, at low temperature (i.e., below 50°C) and short cycles (i.e., the main wash last less than 40 minutes, preferably less than 30 minutes).

According to the last aspect of the invention there is provided the use of the composition of the invention for the removal of burnt-on sugary food soils, such as crème brulee, in automatic dishwashing.

The elements of the composition of the invention described in connexion with the first aspect of the invention apply *mutatis mutandis* to the other aspects of the invention.

### BRIEF DESCRIPTION OF THE DRAWINGS

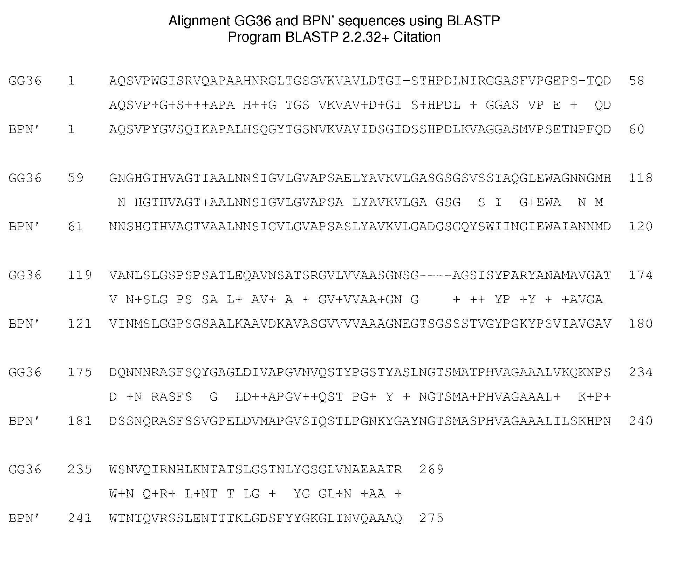

Figure 1 represents alignment of GG36 and BPN' sequences using BLASTP.

### DETAILED DESCRIPTION OF THE INVENTION

The present invention encompasses an automatic dishwashing cleaning composition. The composition is phosphate-free and comprises a protease. The composition provides improved removal of burnt-on sugary food soils, such as crème brûlée. The invention also encompasses methods of automatic dishwashing, involving short cycles and/or low temperatures and the use of the composition of the invention to provide removal of burnt-on sugary food soils, such as crème brûlée, in automatic dishwashing.
As used herein, "dishware" includes cookware and tableware.

The protease of the composition of the invention is herein sometimes referred to as "the protease of the invention". The protease having SEQ ID NO:1 (subtilisin GG36 from Bacillus lentus) is herein sometimes referred to as "the parent protease". The variant protease of the invention is different from proteases with SEQ ID NO:3, SEQ ID NO:4, or SEQ ID NO: 5.

The relatedness between two amino acid sequences or between two nucleotide sequences is described by the parameter "sequence identity".
The term "variant" means a protease comprising a mutation, i.e., a substitution, insertion, and/or deletion, at one or more (e.g., several) positions relative to the parent protease of SEQ ID NO:1. A substitution means replacement of the amino acid occupying a position with a different amino acid; a deletion means removal of the amino acid occupying a position; and an insertion means adding an amino acid adjacent to and immediately following the amino acid occupying a position. The variants of the present invention have at least 90%, preferably at least 95%, more preferably at least 98% identity with the protease of SEQ ID NO: 1.

The term "wild-type" protease means a protease expressed by a naturally occurring microorganism, such as a bacterium, yeast, or filamentous fungus found in nature.

### Enzyme related terminology

### Nomenclature for amino acid modifications

In describing enzyme variants herein, the following nomenclature is used for ease of reference: Original amino acid(s):position(s):substituted amino acid(s).

According to this nomenclature, for instance a glycine to glutamic acid substitution in position 195 is shown as G195E. A deletion of glycine in the same position is shown as G195*, and insertion of an additional amino acid residue such as lysine is shown as G195GK. Where a specific enzyme contains a "deletion" in comparison with other enzyme and an insertion is made in such a position this is indicated as *36D for insertion of an aspartic acid in position 36. Multiple mutations are separated by pluses, i.e.: S99G+V102N, representing serine to glycine and valine to asparagine substitutions in positions 99 and 102 respectively. Where the amino acid in a position (*e.g.* 102) may be substituted by another amino acid selected from a group of amino acids, e.g. the group consisting of N and I, this will be indicated by V102{N,I} representing valine substitution to either asparagine or isoleucine at position 102.
In all cases, the accepted IUPAC single letter or triple letter amino acid abbreviation is employed.

### Protease Amino Acid Numbering

The numbering used in this patent is the BPN' numbering system which is commonly used in the art. An alternative numbering scheme is numbering the specific amino acid sequence of the protease (GG36)-listed as SEQ ID NO:1. For convenience the two different numbering schemes are compared below in Table 1:

**Table 1 - Protease Mutation numbering**

| GG36 numbering (numbering versus SEQ ID NO:1) | Equivalent BPN' numbering of this patent |
|---|---|
| N74D + S85R + G116R + S126L + P127Q + S128A | N76D + S87R + G118R + S128L + P129Q + S130A |
| N74D + S85R + G116R + S126L + P127Q + S128A + S182D + V238R | N76D + S87R + G118R + S128L + P129Q + S130A + S188D + V244R |

Figure 1 shows the alignment of the mature amino acid sequence of *B. lentus* subtilisin GG36, the mature amino acid sequence of *B. amyloliquefaciens* subtilisin BPN'.

### Amino acid identity

The relatedness between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of an enzyme used herein ("invention sequence") and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity. An exact match occurs when the "invention sequence" and the "foreign sequence" have identical amino acid residues in the same positions of the overlap. The length of a sequence is the number of amino acid residues in the sequence.
The term "succinate based compound" and "succinic acid based compound" are used interchangeably herein.

As used herein, articles such as "a" and "an" when used in a claim, are understood to mean one or more of what is claimed or described.
Unless otherwise noted, all component or composition levels are in reference to the active portion of that component or composition, and are exclusive of impurities, for example, residual solvents or by-products, which may be present in commercially available sources of such components or compositions.
All percentages and ratios are calculated by weight unless otherwise indicated. All percentages and ratios are calculated based on the total composition unless otherwise indicated.

### The protease of the invention

The protease of the invention is a variant of a parent protease. The parent protease has the amino acid sequence of SEQ ID NO: 1. The variant protease has at least 90%, preferably at least 95% and more preferably at least 97% identity with the amino acid sequence of SEQ ID NO: 1. The variant protease comprises at least one amino acid residue with a carboxylic acid side chain, i.e. aspartic acid and/or glutamic acid, in the active site loop region from position 95 to 103, preferably from position 96 to 102 and more preferably from position 97 to 101 and especially in position 99 (BPN' numbering). The amino acid residue with a carboxylic acid side chain may be introduced by either substitution or insertion. The protease of the invention excludes proteases having the amino acid sequence of SEQ ID NO: 3, SEQ ID NO: 4 and SEQ ID NO: 5. Preferably, the protease of the invention is selected from the group consisting of:
1) A variant comprising one or more of the following amino acid substitutions: X95{D,E}, X96{D,E}, X97{D,E}, X98{D,E}, X99{D,E}, X100{D,E}, X10 {D,E}, X102{D,E}, X103{D,E} versus SEQ NO:1;
2) A variant comprising one or more of the following amino acid insertions: X95X{D,E}, X96X{D,E}, X97X{D,E}, X98X{D,E}, X99X{D,E}, X100X{D,E}, X101X{D,E}, X102X{D,E}, X103X{D,E} versus SEQ ID 1 NO:1; and
3) A variant comprising one or more of the substitutions listed in 1), in combination with one or more of the insertions listed in 2);
Preferably the protease of the invention and in particular any of the protease variants of 1), 2) and 3) further comprises additional substitutions in one or more of the positions selected from the group consisting of: 3, 4, 9, 15, 22, 27, 61, 68, 76, 87, 96-109, 118, 126-132, 159, 160-168, 170, 175, 181-192, 194, 195, 199, 203-206, 209-222, 245 and 274 versus SEQ ID NO:1.

More preferably, the protease of the invention is selected from the group consisting of:
1) A variant comprising one or more of the following amino acid substitutions: V95{D,E}, L96{D,E}, G97{D,E}, A98{D,E}, S99{D,E}, G100{D,E}, S101{D,E}, G102{D,E}, and S103{D,E} versus SEQ ID NO:1;
2) A variant comprising one or more of the following amino acid insertions: V95V{D,E}, L96L{D,E}, G97G{D,E}, A98A{D,E}, S99S{D,E}, G100G{D,E}, S101S{D,E}, G102G{D,E} and S103S{D,E} versus SEQ ID NO:1;
3) A variant comprising one or more of the substitutions listed in 1) in combination with one or more of the insertions listed in 2); and
4) Any of the protease variants of 1), 2) and 3) further comprising additional substitutions in one or more of the positions selected from the group consisting of: 3, 4, 9, 15, 22, 27, 61, 68, 76, 87, 96-109, 118, 126-132, 159, 160-168, 170, 175, 181-192, 194, 195, 199, 203-206, 209-222, 245 and 274 versus SEQ ID NO:1.

More preferably, the protease of the invention is selected from the group consisting of:
1) A variant comprising one or more of the following amino acid substitutions: V95{D,E}, L96{D,E}, G97{D,E}, A98{D,E}, S99{D,E}, G100{D,E}, S101{D,E}, G102{D,E}, S103{D,E} versus SEQ ID NO:1;
2) A variant comprising one or more of the following amino acid insertions: V95V{D,E}, L96L{D,E}, G97G{D,E}, A98A{D,E}, S99S{D,E}, G100G{D,E}, S101S{D,E}, G102G{D,E}, S103S{D,E} versus SEQ ID NO:1;
3) A variant comprising one or more of the substitutions listed in 1),in combination with one or more of the insertions listed in 2);
4) Any of the protease variants of 1), 2) and 3) further comprising one or more additional substitutions selected from the group consisting of: S3T; V4I; S9R; A15T; V68A; N76D; S87{N,R}; S99A; S101{G,M,R}; S103A; V104{I,N}; G118{R,V}; S128L; P129Q; S130A; G159{D,S}; Y167A; R170S; A194P; G195E; V205I; N218{D,G,V}; M222{A,S}; and Q245R versus SEQ ID NO:1.

More preferably, the protease of the invention is selected from the group consisting of:
1) A variant comprising one or more of the following amino acid substitutions: V95{D,E}, L96{D,E}, G97{D,E}, A98{D,E}, S99{D,E}, G100{D,E}, S101{D,E}, G102{D,E} and S103{D,E} versus SEQ ID NO:1;
2) A variant comprising one or more of the following amino acid insertions: V95V{D,E}, L96L{D,E}, G97G{D,E}, A98A{D,E}, S99S{D,E}, G100G{D,E}, S101S{D,E}, G102G{D,E} and S103S{D,E} versus SEQ ID NO:1;
3) A variant comprising one or more of the substitutions listed in 1),in combination with one or more of the insertions listed in 2);
4) Any of the protease variants of 1), 2) and 3) further comprising one of the following additional substitutions versus SEQ ID NO: 1:
   (i) G195E + M222A;
   (ii) M222S;
   (iii) N76D + S87R + G118R+ S128L+P129Q+S130A;
   (iv) N76D + S103A + V104I;
   (v) S3T+S103A+V104I+G159S+V205I;
   (vi) S9R+A15T+V68A+N218D;
   (vii) S87N + G118V+S128L+P129Q+S130A;
   (viii) S87N +S101G +V104N;
   (ix) S87N;
   (x) S101M+ G118V+ S128L+P129Q+S130A;
   (xi) S101G+S103A+V104I+Q245R;
   (xii) S101R+S103A+V104I+G159S;
   (xiii) S101G+V104N;
   (xiv) S103A + V104I+G159D;
   (xv) S103A + V104I;
   (xvi) V68A+S101G+V104N; and
   (xvii) Y167A + R170S + A194P

Most preferably, the protease of the invention is selected from the group consisting of:
1) A variant comprising one or more of the following amino acid substitutions: V95{D,E}, L96{D,E}, G97{D,E}, A98{D,E}, S99{D,E}, G100{D,E}, S101{D,E}, G102{D,E} and S103{D,E} versus SEQ ID NO:1;
2) A variant comprising one or more of the following amino acid insertions: V95V{D,E}, L96L{D,E}, G97G{D,E}, A98A{D,E}, S99S{D,E}, G100G{D,E}, S101S{D,E}, G102G{D,E} and S103S{D,E} versus SEQ ID NO:1;
3) A variant comprising one or more of the substitutions listed in 1),in combination with one or more of the insertions listed in 2);
4) Any of the protease variants of 1), 2) and 3) further comprising additional substitutions in two or more of the following positions: 3, 4, 9, 15, 22, 61, 68, 76, 87, 96-109, 118, 126-132, 159, 160-166, 168, 175, 181-192, 195, 199, 203-205, 209-222, 245 and 274 vs SEQ ID NO:1, specially one of the following additional substitutions versus SEQ ID NO:1:
   (i) G195E + M222A;
   (ii) N76D + S87R + G118R+ S128L+P129Q+S130A;
   (iii) S3T+S103A+V104I+G159S+V205I;
   (iv) S9R+A15T+V68A+N218D;
   (v) S87N + G118V+S128L+P129Q+S130A;
   (vi) S101M+ G118V+ S128L+P129Q+S130A;
   (vii) S101G+S103A+V104I+Q245R;
   (viii) S101R+S103A+V104I+G159S;
   (ix) S103A + V104I+G159D; and
   (x) V68A+S101G+V104N;

Preferred levels of the variant in the composition of the invention include from about 0.05 to about 2 mg of active protease per gram of the composition.

### Automatic dishwashing cleaning composition

The automatic dishwashing cleaning composition of the invention can be in any physical form. It can be a loose powder, a gel or presented in unit dose form. Preferably it is in unit dose form, unit dose forms include pressed tablets and water-soluble packs. The automatic dishwashing cleaning composition of the invention is preferably presented in unit-dose form and it can be in any physical form including solid, liquid and gel form. The composition of the invention is very well suited to be presented in the form of a multi-compartment pack, more in particular a multi-compartment pack comprising compartments with compositions in different physical forms, for example a compartment comprising a composition in solid form and another compartment comprising a composition in liquid form. The composition is preferably enveloped by a water-soluble film such as polyvinyl alcohol. Especially preferred are compositions in unit dose form wrapped in a polyvinyl alcohol film having a thickness of less than 100 µm. The detergent composition of the invention weighs from about 8 to about 25 grams, preferably from about 10 to about 20 grams. This weight range fits comfortably in a dishwasher dispenser. Even though this range amounts to a low amount of detergent, the detergent has been formulated in a way that provides all the benefits mentioned herein above.

The composition is phosphate free. By "phosphate-free" is herein understood that the composition comprises less than 1%, preferably less than 0.1% by weight of the composition of phosphate.

The composition of the invention is phosphate-free and preferably comprises an organic complexing agent and preferably a dispersant polymer. For the purpose of this invention a "complexing agent" is a compound capable of binding polyvalent ions such as calcium, magnesium, lead, copper, zinc, cadmium, mercury, manganese, iron, aluminium and other cationic polyvalent ions to form a water-soluble complex.
The organic complexing agent is preferably selected from the group consisting of citric acid, its salts and derivatives thereof, methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, ASDA (L-Aspartic acid N, N-diacetic acid tetrasodium salt), its salts and derivatives thereof its salts and derivatives thereof and mixtures thereof. Especially preferred complexing agents for use herein are selected from the group consisting of citric acid and its salts and MGDA and its salts, especially preferred for use herein are sodium citrate and the tri-sodium salt of MGDA. Preferably, the complexing agent comprises the tri-sodium salt of MGDA. Preferably the composition comprises a dispersant polymer, more preferably a sulfonated polymer, even more preferably a sulfonated polymer comprising 2-acrylamido-2-methylpropane sulfonic acid monomer. Preferably, the composition of the invention comprises a high level of complexing agent. Preferably the composition comprises at least 15%, more preferably at least 20%, more preferably at least 25% of complexing agent by weight of the composition. The composition preferably comprises less than 70% of complexing agent by weight of the composition. In a preferred embodiment, the composition comprises from 25% to 50% by weight of the composition of the tri-sodium salt of MGDA. These compositions are very good for the removal of tough food soils, such as cooked-, baked-on and burnt-on soils. In another preferred embodiment, the composition comprises from 15% to 50% by weight of the composition of the sodium citrate and the tri-sodium salt of MGDA. More preferably, the sodium citrate and the tri-sodium salt of MGDA are in weight ratio of from 0.5:1 to 2:1. The protease of the invention presents good stability in wash and in storage.

### Dispersant polymer

A dispersant polymer can be used in any suitable amount from about 0.1 to about 20%, preferably from 0.2 to about 15%, more preferably from 0.3 to 8% by weight of the composition.

The dispersant polymer is capable to suspend calcium or calcium carbonate in an automatic dishwashing process.

The dispersant polymer has a calcium binding capacity within the range between 30 to 250 mg of Ca/g of dispersant polymer, preferably between 35 to 200 mg of Ca/g of dispersant polymer, more preferably 40 to 150 mg of Ca/g of dispersant polymer at 25°C. In order to determine if a polymer is a dispersant polymer within the meaning of the invention, the following calcium binding-capacity determination is conducted in accordance with the following instructions:
Calcium binding capacity test method

The calcium binding capacity referred to herein is determined via titration using a pH/ion meter, such as the Meettler Toledo SevenMulti^{™} bench top meter and a PerfectION^{™} comb Ca combination electrode. To measure the binding capacity a heating and stirring device suitable for beakers or tergotometer pots is set to 25 °C, and the ion electrode with meter are calibrated according to the manufacturer's instructions. The standard concentrations for the electrode calibration should bracket the test concentration and should be measured at 25 °C. A stock solution of 1000 mg/g of Ca is prepared by adding 3.67 g of CaCl₂-2H₂O into 1 L of deionised water, then dilutions are carried out to prepare three working solutions of 100 mL each, respectively comprising 100 mg/g, 10 mg/g, and 1 mg/g concentrations of Calcium. The 100 mg Ca/g working solution is used as the initial concentration during the titration, which is conducted at 25 °C. The ionic strength of each working solution is adjusted by adding 2.5 g/L of NaCl to each. The 100 mL of 100 mg Ca/g working solution is heated and stirred until it reaches 25 °C. The initial reading of Calcium ion concentration is conducted at when the solution reaches 25 °C using the ion electrode. Then the test polymer is added incrementally to the calcium working solution (at 0.01 g/L intervals) and measured after 5 minutes of agitation following each incremental addition. The titration is stopped when the solution reaches 1 mg/g of Calcium. The titration procedure is repeated using the remaining two calcium concentration working solutions. The binding capacity of the test polymer is calculated as the linear slope of the calcium concentrations measured against the grams/L of test polymer that was added.

The dispersant polymer preferably bears a negative net charge when dissolved in an aqueous solution with a pH greater than 6.

The dispersant polymer can bear also sulfonated carboxylic esters or amides, in order to increase the negative charge at lower pH and improve their dispersing properties in hard water. The preferred dispersant polymers are selected from the group consisting of carboxylated polymers free of sulfonated groups, sulfonated polymers (i.e., polymers comprising sulfonated monomers) and mixtures thereof.

Preferably, the dispersant polymers are sulfonated derivatives of polycarboxylic acids and may comprise two, three, four or more different monomer units. The preferred copolymers contain:

At least one structural unit derived from a carboxylic acid monomer having the general formula (III): 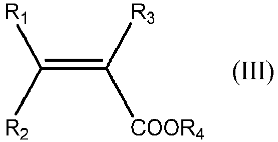 wherein R₁ to R₃ are independently selected from hydrogen, methyl, linear or branched saturated alkyl groups having from 2 to 12 carbon atoms, linear or branched mono or polyunsaturated alkenyl groups having from 2 to 12 carbon atoms, alkyl or alkenyl groups as aforementioned substituted with -NH2 or -OH, or -COOH, or COOR₄, where R₄ is selected from hydrogen, alkali metal, or a linear or branched, saturated or unsaturated alkyl or alkenyl group with 2 to 12 carbons;

Preferred carboxylic acid monomers include one or more of the following: acrylic acid, maleic acid, maleic anhydride, itaconic acid, citraconic acid, 2-phenylacrylic acid, cinnamic acid, crotonic acid, fumaric acid, methacrylic acid, 2-ethylacrylic acid, methylenemalonic acid, or sorbic acid. Acrylic and methacrylic acids being more preferred.

Optionally, one or more structural units derived from at least one nonionic monomer having the general formula (IV):

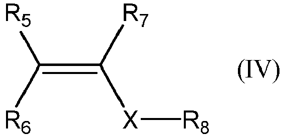

Wherein R₅ to R₇ are independently selected from hydrogen, methyl, phenyl or hydroxyalkyl groups containing 1 to 6 carbon atoms, and can be part of a cyclic structure, X is an optionally present spacer group which is selected from -CH₂-, -COO-, -CONH- or -CONR₈-, and R₈ is selected from linear or branched, saturated alkyl radicals having 1 to 22 carbon atoms or unsaturated, preferably aromatic, radicals having from 6 to 22 carbon atoms.

Preferred non-ionic monomers include one or more of the following: butene, isobutene, pentene, 2-methylpent-1-ene, 3-methylpent-1-ene, 2,4,4-trimethylpent-1-ene, 2,4,4-trimethylpent-2-ene, cyclopentene, methylcyclopentene, 2-methyl-3-methyl-cyclopentene, hexene, 2,3-dimethylhex-1-ene, 2,4-dimethylhex-1-ene, 2,5-dimethylhex-1-ene, 3,5-dimethylhex-1-ene, 4,4-dimethylhex-1-ene, cyclohexene, methylcyclohexene, cycloheptene, alpha olefins having 10 or more carbon atoms such as, dec-1-ene, dodec-1-ene, hexadec-1-ene, octadec-1-ene and docos-1-ene, preferred aromatic monomers are styrene, alpha methylstyrene, 3-methylstyrene, 4-dodecylstyrene, 2-ethyl-4-bezylstyrene, 4-cyclohexylstyrene, 4-propylstyrol, 1-vinylnaphtalene, 2-vinylnaphtalene; preferred carboxylic ester monomers are methyl (meth)acrylate, ethyl (meth)acrylate, propyl (meth)acrylate, t-butyl (meth)acrylate, pentyl (meth)acrylate, hexyl (meth)acrylate, 2-ethylhexyl (meth)acrylate, octyl (meth)acrylate, lauryl (meth)acrylate, stearyl (meth)acrylate and behenyl (meth)acrylate; preferred amides are N-methyl acrylamide, N-ethyl acrylamide, N-t-butyl acrylamide, N-2-ethylhexyl acrylamide, N-octyl acrylamide, N-lauryl acrylamide, N-stearyl acrylamide, N-behenyl acrylamide.
and at least one structural unit derived from at least one sulfonic acid monomer having the general formula (V) and (VI): 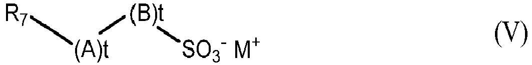 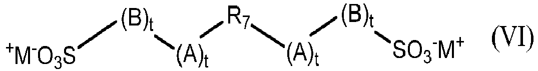 wherein R₇ is a group comprising at least one sp2 bond, A is O, N, P, S, an amido or ester linkage, B is a mono- or polycyclic aromatic group or an aliphatic group, each t is independently 0 or 1, and M+ is a cation. In one aspect, R₇ is a C2 to C6 alkene. In another aspect, R7 is ethene, butene or propene.

Preferred sulfonated monomers include one or more of the following: 1-acrylamido-1-propanesulfonic acid, 2-acrylamido-2-propanesulfonic acid, 2-acrylamido-2-methyl-1-propanesulfonic acid, 2-methacrylamido-2-methyl-1-propanesulfonic acid, 3- methacrylamido-2-hydroxy-propanesulfonic acid, allylsulfonic acid, methallylsulfonic acid, allyloxybenzenesulfonic acid, methallyloxybenzenesulfonic acid, 2-hydroxy-3- (2-propenyloxy) propanesulfonic acid, 2-methyl-2-propen-1-sulfonic acid, styrenesulfonic acid, vinylsulfonic acid, 3-sulfopropyl, 3-sulfo-propylmethacrylate, sulfomethacrylamide, sulfomethylmethacrylamide and mixtures of said acids or their water-soluble salts.

Preferably, the polymer comprises the following levels of monomers: from about 40 to about 90%, preferably from about 60 to about 90% by weight of the polymer of one or more carboxylic acid monomer; from about 5 to about 50%, preferably from about 10 to about 40% by weight of the polymer of one or more sulfonic acid monomer; and optionally from about 1% to about 30%, preferably from about 2 to about 20% by weight of the polymer of one or more non-ionic monomer. An especially preferred polymer comprises about 70% to about 80% by weight of the polymer of at least one carboxylic acid monomer and from about 20% to about 30% by weight of the polymer of at least one sulfonic acid monomer.

In the polymers, all or some of the carboxylic or sulfonic acid groups can be present in neutralized form, i.e. the acidic hydrogen atom of the carboxylic and/or sulfonic acid group in some or all acid groups can be replaced with metal ions, preferably alkali metal ions and in particular with sodium ions.

The carboxylic acid is preferably (meth)acrylic acid. The sulfonic acid monomer is preferably 2-acrylamido-2-propanesulfonic acid (AMPS).

Preferred commercial available polymers include: Alcosperse 240, Aquatreat AR 540 and Aquatreat MPS supplied by Alco Chemical; Acumer 3100, Acumer 2000, Acusol 587G and Acusol 588G supplied by Rohm & Haas; Goodrich K-798, K-775 and K-797 supplied by BF Goodrich; and ACP 1042 supplied by ISP technologies Inc. Particularly preferred polymers are Acusol 587G and Acusol 588G supplied by Rohm & Haas.

Suitable dispersant polymers include anionic carboxylic polymer of low molecular weight. They can be homopolymers or copolymers with a weight average molecular weight of less than or equal to about 200,000 g/mol, or less than or equal to about 75,000 g/mol, or less than or equal to about 50,000 g/mol, or from about 3,000 to about 50,000 g/mol, preferably from about 5,000 to about 45,000 g/mol. The dispersant polymer may be a low molecular weight homopolymer of polyacrylate, with an average molecular weight of from 1,000 to 20,000, particularly from 2,000 to 10,000, and particularly preferably from 3,000 to 5,000.

The dispersant polymer may be a copolymer of acrylic with methacrylic acid, acrylic and/or methacrylic with maleic acid, and acrylic and/or methacrylic with fumaric acid, with a molecular weight of less than 70,000. Their molecular weight ranges from 2,000 to 80,000 and more preferably from 20,000 to 50,000 and in particular 30,000 to 40,000 g/mol. and a ratio of (meth)acrylate to maleate or fumarate segments of from 30:1 to 1:2.

The dispersant polymer may be a copolymer of acrylamide and acrylate having a molecular weight of from 3,000 to 100,000, alternatively from 4,000 to 20,000, and an acrylamide content of less than 50%, alternatively less than 20%, by weight of the dispersant polymer can also be used. Alternatively, such dispersant polymer may have a molecular weight of from 4,000 to 20,000 and an acrylamide content of from 0% to 15%, by weight of the polymer.

Dispersant polymers suitable herein also include itaconic acid homopolymers and copolymers. Alternatively, the dispersant polymer can be selected from the group consisting of alkoxylated polyalkyleneimines, alkoxylated polycarboxylates, polyethylene glycols, styrene co-polymers, cellulose sulfate esters, carboxylated polysaccharides, amphiphilic graft copolymers and mixtures thereof.

### Bleach

The composition of the invention preferably comprises from about 1 to about 20%, more preferably from about 5 to about 18%, even more preferably from about 8 to about 15% of bleach by weight of the composition.
Inorganic and organic bleaches are suitable for use herein. Inorganic bleaches include perhydrate salts such as perborate, percarbonate, perphosphate, persulfate and persilicate salts. The inorganic perhydrate salts are normally the alkali metal salts. The inorganic perhydrate salt may be included as the crystalline solid without additional protection. Alternatively, the salt can be coated. Suitable coatings include sodium sulphate, sodium carbonate, sodium silicate and mixtures thereof. Said coatings can be applied as a mixture applied to the surface or sequentially in layers.
Alkali metal percarbonates, particularly sodium percarbonate is the preferred bleach for use herein. The percarbonate is most preferably incorporated into the products in a coated form which provides in-product stability.
Potassium peroxymonopersulfate is another inorganic perhydrate salt of utility herein.
Typical organic bleaches are organic peroxyacids, especially dodecanediperoxoic acid, tetradecanediperoxoic acid, and hexadecanediperoxoic acid. Mono- and diperazelaic acid, mono-and diperbrassylic acid are also suitable herein. Diacyl and Tetraacylperoxides, for instance dibenzoyl peroxide and dilauroyl peroxide, are other organic peroxides that can be used in the context of this invention.
Further typical organic bleaches include the peroxyacids, particular examples being the alkylperoxy acids and the arylperoxy acids. Preferred representatives are (a) peroxybenzoic acid and its ring-substituted derivatives, such as alkylperoxybenzoic acids, but also peroxy-α-naphthoic acid and magnesium monoperphthalate, (b) the aliphatic or substituted aliphatic peroxy acids, such as peroxylauric acid, peroxystearic acid, ε-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2-decyldiperoxybutane-1,4-dioic acid, N,N-terephthaloyldi(6-aminopercaproic acid).

### Bleach Activators

Bleach activators are typically organic peracid precursors that enhance the bleaching action in the course of cleaning at temperatures of 60° C and below. Bleach activators suitable for use herein include compounds which, under perhydrolysis conditions, give aliphatic peroxoycarboxylic acids having preferably from 1 to 12 carbon atoms, in particular from 2 to 10 carbon atoms, and/or optionally substituted perbenzoic acid. Suitable substances bear O-acyl and/or N-acyl groups of the number of carbon atoms specified and/or optionally substituted benzoyl groups. Preference is given to polyacylated alkylenediamines, in particular tetraacetylethylenediamine (TAED), acylated triazine derivatives, in particular 1,5-diacetyl-2,4-dioxohexahydro-1,3,5-triazine (DADHT), acylated glycolurils, in particular tetraacetylglycoluril (TAGU), N-acylimides, in particular N-nonanoylsuccinimide (NOSI), acylated phenolsulfonates, in particular n-nonanoyl- or isononanoyloxybenzenesulfonate (n- or iso-NOBS), decanoyloxybenzoic acid (DOBA), carboxylic anhydrides, in particular phthalic anhydride, acylated polyhydric alcohols, in particular triacetin, ethylene glycol diacetate and 2,5-diacetoxy-2,5-dihydrofuran and also triethylacetyl citrate (TEAC). If present the composition of the invention comprises from 0.01 to 5, preferably from 0.2 to 2% by weight of the composition of bleach activator, preferably TAED.

### Bleach Catalyst

The composition herein preferably contains a bleach catalyst, preferably a metal containing bleach catalyst. More preferably the metal containing bleach catalyst is a transition metal containing bleach catalyst, especially a manganese or cobalt-containing bleach catalyst.
Bleach catalysts preferred for use herein include manganese triazacyclononane and related complexes; manganese oxalate, Co, Cu, Mn and Fe bispyridylamine and related complexes; and pentamine acetate cobalt(III) and related complexes.
Preferably the composition of the invention comprises from 0.001 to 0.5, more preferably from 0.002 to 0.05% of bleach catalyst by weight of the composition. Preferably the bleach catalyst is a manganese bleach catalyst.

### Inorganic builder

The composition of the invention preferably comprises an inorganic builder. Suitable inorganic builders are selected from the group consisting of carbonate, silicate and mixtures thereof. Especially preferred for use herein is sodium carbonate. Preferably the composition of the invention comprises from 5 to 50%, more preferably from 10 to 40% and especially from 15 to 30% of sodium carbonate by weight of the composition.

### Surfactant

Surfactants suitable for use herein include non-ionic surfactants, preferably the compositions are free of any other surfactants. Traditionally, non-ionic surfactants have been used in automatic dishwashing for surface modification purposes in particular for sheeting to avoid filming and spotting and to improve shine. It has been found that non-ionic surfactants can also contribute to prevent redeposition of soils.
Preferably the composition of the invention comprises a non-ionic surfactant or a non-ionic surfactant system, more preferably the non-ionic surfactant or a non-ionic surfactant system has a phase inversion temperature, as measured at a concentration of 1% in distilled water, between 40 and 70°C, preferably between 45 and 65°C. By a "non-ionic surfactant system" is meant herein a mixture of two or more non-ionic surfactants. Preferred for use herein are non-ionic surfactant systems. They seem to have improved cleaning and finishing properties and better stability in product than single non-ionic surfactants.

Phase inversion temperature is the temperature below which a surfactant, or a mixture thereof, partitions preferentially into the water phase as oil-swollen micelles and above which it partitions preferentially into the oil phase as water swollen inverted micelles. Phase inversion temperature can be determined visually by identifying at which temperature cloudiness occurs. The phase inversion temperature of a non-ionic surfactant or system can be determined as follows: a solution containing 1% of the corresponding surfactant or mixture by weight of the solution in distilled water is prepared. The solution is stirred gently before phase inversion temperature analysis to ensure that the process occurs in chemical equilibrium. The phase inversion temperature is taken in a thermostable bath by immersing the solutions in 75 mm sealed glass test tube. To ensure the absence of leakage, the test tube is weighed before and after phase inversion temperature measurement. The temperature is gradually increased at a rate of less than 1°C per minute, until the temperature reaches a few degrees below the pre-estimated phase inversion temperature. Phase inversion temperature is determined visually at the first sign of turbidity.
Suitable nonionic surfactants include: i) ethoxylated non-ionic surfactants prepared by the reaction of a monohydroxy alkanol or alkyphenol with 6 to 20 carbon atoms with preferably at least 12 moles particularly preferred at least 16 moles, and still more preferred at least 20 moles of ethylene oxide per mole of alcohol or alkylphenol; ii) alcohol alkoxylated surfactants having a from 6 to 20 carbon atoms and at least one ethoxy and propoxy group. Preferred for use herein are mixtures of surfactants i) and ii).
Another suitable non-ionic surfactants are epoxy-capped poly(oxyalkylated) alcohols represented by the formula:

R1O[CH2CH(CH3)O]x[CH2CH2O]y[CH2CH(OH)R2] (I)

wherein R1 is a linear or branched, aliphatic hydrocarbon radical having from 4 to 18 carbon atoms; R2 is a linear or branched aliphatic hydrocarbon radical having from 2 to 26 carbon atoms; x is an integer having an average value of from 0.5 to 1.5, more preferably about 1; and y is an integer having a value of at least 15, more preferably at least 20.

Preferably, the surfactant of formula I, at least about 10 carbon atoms in the terminal epoxide unit [CH2CH(OH)R2]. Suitable surfactants of formula I, according to the present invention, are Olin Corporation's POLY-TERGENT® SLF-18B nonionic surfactants, as described, for example, in WO 94/22800, published October 13, 1994 by Olin Corporation.

### Enzymes

### Other proteases

The composition of the invention can comprise a protease in addition to the protease of the invention. A mixture of two or more proteases can contribute to an enhanced cleaning across a broader temperature, cycle duration, and/or substrate range, and provide superior shine benefits, especially when used in conjunction with an anti-redeposition agent and/or a sulfonated polymer.
Suitable proteases for use in combination with the protease of the invention include metalloproteases and serine proteases, including neutral or alkaline microbial serine proteases, such as subtilisins (EC 3.4.21.62). Suitable proteases include those of animal, vegetable or microbial origin. Microbial origin is preferred. Chemically or genetically modified mutants are included. The protease may be a serine protease, preferably an alkaline microbial protease or a chymotrypsin or trypsin-like protease. Examples of neutral or alkaline proteases include:
(a) subtilisins (EC 3.4.21.62), especially those derived from Bacillus, such as Bacillus sp., B. lentus, B. alkalophilus, B. subtilis, B. amyloliquefaciens, B. pumilus , B. gibsonii, and B. akibaii described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569.
(b) trypsin-like or chymotrypsin-like proteases, such as trypsin (e.g., of porcine or bovine origin), the Fusarium protease described in WO 89/06270 and the chymotrypsin proteases derived from Cellumonas described in WO 05/052161 and WO 05/052146.
(c) metalloproteases, especially those derived from Bacillus amyloliquefaciens decribed in WO 07/044993A2; ; from Bacillus, Brevibacillus, Thermoactinomyces, Geobacillus, Paenibacillus, Lysinibacillus or Streptomyces spp. described in WO2014194032, WO2014194054 and WO2014194117; from Kribella alluminosa described in WO2015193488; and from Streptomyces and Lysobacter described in WO2016075078.

Especially preferred additional proteases for the detergent of the invention are polypeptides demonstrating at least 70%, preferably at least 90%, more preferably at least 95%, even more preferably at least 99% and especially 100% identity with the wild-type subtilisin 309 from Bacillus lentus shown in SEQ ID NO:1, comprising mutations in one or more, preferably two or more and more preferably three or more of the following positions, using the BPN' numbering system:
3, 4, 9, 15, 32, 33, 49-56, 60-68, 76, 87, 96-109, 118, 125-135, 152, 154-159, 160-168, 170, 175, 181-192, 194, 195, 199, 203-206, 209-222, 244, 245, 248, 274

Most preferably the additional protease is selected from the group comprising the below mutations (BPN' numbering system) versus GG36 parent protease shown in SEQ ID NO:1..
(i) G118V + S128L + P129Q + S130A;
(ii) G195E + M222A;
(iii) M222S;
(iv) N76D+S87R+G118R+S128L+P129Q+S130A+S188D+V244R
(v) N76D+S87R+G118R+S128L+P129Q+S130A+S188D+N248R
(vi) N76D + S87R + G118R+ S128L+P129Q+S130A;
(vii) N76D + S103A + V104I;
(viii) S3T+S103A+V104I+G159S+V205I;
(ix) S9R+A15T+V68A+N218D;
(x) S87N +S101G +V104N;
(xi) S87N + G118V+S128L+P129Q+S130A;
(xii) S87N;
(xiii) S99A + S99SD;
(xiv) S99D + S101R + S103A + V104I +G159S;
(xv) S3T + V4I + S99D + S101R + S103A + V104I +G159S;
(xvi) S101M+ G118V+ S128L+P129Q+S130A;
(xvii) (vxii) S101R + S103A + V104I + G159S;
(xviii) S101G + S103A + V104 + Q245R;
(xix) S101G+V104N;
(xx) S103A + V104I + G159D;
(xxi) S103A + V104I;
(xxii) V68A+S101G+V104N; and
(xxiii) Y167A + R170S + A194P

Suitable commercially available protease enzymes include those sold under the trade names Alcalase®, Savinase®, Primase®, Durazym®, Polarzyme®, Kannase®, Liquanase®, Neutrase®, Everlase®, Esperase®, and Blaze by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/ Kemira, namely BLAP and BLAP variants.

Especially preferred for use herein in combination with the variant protease of the invention are commercial proteases selected from the group consisting of Properase®, Purafect®, Ultimase®, Everlase®, Savinase®, Excellase®, FN3®, Blaze, BLAP and BLAP variants.

Preferred levels of protease in the composition of the invention include from about 0.05 to about 0.5, more preferably from about 0.025 to about 0.35 and especially from about 0.05 to about 0.3 mg of active protease per grams of the composition.

### Amylases

In addition to the protease of the invention the composition of the invention can comprise amylases. A preferred alkaline amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCIB 12289, NCIB 12512, NCIB 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) the variants described in US 5,856,164 and WO99/23211, WO 96/23873, WO00/60060 and WO 06/002643, especially the variants with one or more substitutions in the following positions versus the AA560 SEQ ID No. 3:
   9, 26, 30, 33, 82, 37, 106, 118, 128, 133, 149, 150, 160, 178, 182, 186, 193, 195, 202, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482, 484, preferably that also contain the deletions of D183* and G184*.
(b) variants exhibiting at least 90% identity with the wild-type enzyme from Bacillus SP722 (SEQ ID No. 4 in WO06/002643, p.7-9 of sequence listings), especially variants with deletions in the 183 and 184 positions and variants described in WO00/60060, which is incorporated herein by reference.
(c) variants exhibiting at least 95% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one of M202L or M202T mutations.
(d) variants exhibiting at least 60% amino acid sequence identity with the "CspAmy2 α-amylase" from *Cytophaga sp.* (SEQ ID NO:9 in WO2014164777), especially variants with one or more of the following mutations E187P, I203Y, G476K, R458N, T459S and/or D460T.
(e) variants exhibiting at least 60% amino acid sequence identity with the "PcuAmyl α-amylase" from *Paenibacillus curdlanolyticus* YK9 (SEQ ID NO:3 in WO2014099523).
(f) variants exhibiting at least 85% identity with AmyE from *Bacillus subtilis* (SEQ ID NO:1 in WO2009149271).

Suitable commercially available alpha-amylases include DURAMYL®, LIQUEZYME®, TERMAMYL®, TERMAMYL ULTRA®, NATALASE®, EVEREST®, SUPRAMYL®, STAINZYME®, STAINZYME PLUS®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria, RAPIDASE® , PURASTAR®, ENZYSIZE®, OPTISIZE HT PLUS®, POWERASE®, EXCELLENZ^{™} S series, including EXCELLENZ^{™} S 1000 and EXCELLENZ^{™} S 2000 and PURASTAR OXAM® (DuPont Industrial Biosciences., Palo Alto, California) and KAM® (Kao, 14-10 Nihonbashi Kayabacho, 1-chome, Chuo-ku Tokyo 103-8210, Japan). Amylases especially preferred for use herein include NATALASE®, STAINZYME®, STAINZYME PLUS®, EXCELLENZ^{™} S 1000, EXCELLENZ^{™} S2000 and mixtures thereof.
Preferably, the composition of the invention comprises at least 0.005 mg, preferably from about 0.0025 to about 0.025, more preferably from about 0.05 to about 0.3, especially from about 0.01 to about 0.25 mg of active amylase.
Preferably, the protease and/or amylase of the composition of the invention are in the form of granulates, the granulates comprise more than 29% of sodium sulfate by weight of the granulate and/or the sodium sulfate and the active enzyme (protease and/or amylase) are in a weight ratio of between 3:1 and 100:1 or preferably between 4:1 and 30:1 or more preferably between 5:1 and 20:1.

### Crystal growth inhibitor

Crystal growth inhibitors are materials that can bind to calcium carbonate crystals and prevent further growth of species such as aragonite and calcite.

Especially preferred crystal growth inhibitor for use herein is HEDP (1-hydroxyethylidene 1,1-diphosphonic acid). Preferably, the composition of the invention comprises from 0.01 to 10%, more preferably from 0.1 to 8% and especially from 1 to 8% of a crystal growth inhibitor by weight of the product, preferably HEDP. Compositions comprising HEDP, in particular from 1 to 8% provide good shine, in particular on plastic items.

### Metal Care Agents

Metal care agents may prevent or reduce the tarnishing, corrosion or oxidation of metals, including aluminium, stainless steel and non-ferrous metals, such as silver and copper. Preferably the composition of the invention comprises from 0.1 to 5%, more preferably from 0.2 to 4% and especially from 0.3 to 3% by weight of the product of a metal care agent, preferably the metal care agent is benzo triazole (BTA).

### Glass Care Agents

Glass care agents protect the appearance of glass items during the dishwashing process. Preferably the composition of the invention comprises from 0.1 to 5%, more preferably from 0.2 to 4% and specially from 0.3 to 3% by weight of the composition of a metal care agent, preferably the glass care agent is a zinc containing material, specially hydrozincite.
The automatic dishwashing composition of the invention preferably has a pH as measured in 1% weight/volume aqueous solution in distilled water at 25°C of from about 9.5 to about 12, more preferably from about 10 to less than about 11.5 and especially from about 10.5 to about 11.5. The automatic dishwashing composition of the invention preferably has a reserve alkalinity of from about 10 to about 20, more preferably from about 12 to about 18 at a pH of 9.5 as measured in NaOH with 100 grams of product at 20°C.

A preferred automatic dishwashing composition of the invention comprises:
a) from 0.05 to 2 mg/g of the composition of the variant protease ;
b) from 0.025 to 0.3 mg/g of the composition of amylase;
c) from 5 to 20% by weight of the composition of bleach;
d) from 10 to 40% by weight of the composition of a complexing agent, preferably more than 20% of a salt of MGDA or a mixture of a salt of citric acid and a salt of MGDA;
e) from 1 to 10% by weight of the composition of a dispersant polymer; and optionally
f) from 0.5 to 8% by weight of the composition of a crystal growth inhibitor, preferably HEDP.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A phosphate-free automatic dishwashing cleaning composition comprising a protease wherein the protease is a variant of a parent protease having the amino acid sequence of SEQ ID NO:1 and the variant protease has at least 90% identity with the amino acid sequence of SEQ ID NO:1 wherein the protease comprises at least one amino acid residue with a carboxylic acid side chain in the active site loop region from position 95 to 103 (BPN' numbering) with the proviso that the variant protease does not have 100% identity with SEQ ID NO:3, SEQ ID NO:4, or SEQ ID NO: 5.

2. A composition according to claim 1 wherein the variant comprises one or more of the amino acid substitutions selected from the group consisting of X95 {D,E}, X96{D,E}, X97{D,E}, X98{D,E}, X99{D,E}, X100{D,E}, X101{D,E}, X102{D,E} and X103{D,E} versus SEQ ID NO:1.

3. A composition according to claim 1 wherein the variant comprises one or more of the amino acid insertions selected from the group consisting of X95X{D,E}, X96X{D,E}, X97X{D,E}, X98X{D,E}, X99X{D,E}, X100X{D,E}, X101X{D,E}, X102X{D,E}and X103X{D,E} versus SEQ ID NO:1.

4. A composition according to claim 1 wherein the variant comprises
i) one or more of the amino acid substitutions selected from the group consisting of X95{D,E}, X96{D,E}, X97{D,E}, X98{D,E}, X99{D,E}, X100{D,E}, X101 {D,E}, X102{D,E} and X103{D,E} versus SEQ ID NO:1, in combination with
ii) one or more of the insertions selected from the group consisting of X95X{D,E}, X96X{D,E}, X97X{D,E}, X98X{D,E}, X99X{D,E}, X100X{D,E}, X101X{D,E}, X102X{D,E} and X103X{D,E} versus SEQ ID NO:1.

5. A composition according to any of the preceding claims wherein the variant further comprises substitutions in one or more of the positions selected from the group consisting of 3, 4, 9, 15, 22, 27, 61, 68, 76, 87, 96-109, 118, 126-132, 159, 160-168, 170, 175, 181-192, 194, 195, 199, 203-206, 209-222, 245 and 274 versus SEQ ID NO:1.

6. A composition according to any of the preceding claims wherein the protease further comprises additional amino acid substitutions versus SEQ ID NO: 1:
(i) G195E + M222A;
(ii) M222S;
(iii) N76D + S87R + G118R+ S128L+P129Q+S130A;
(iv) N76D + S103A + V104I;
(v) S3T+S103A+V104I+G159S+V205I;
(vi) S9R+A15T+V68A+N218D;
(vii) S87N + G118V+S128L+P129Q+S130A;
(viii) S87N +S101G +V104N;
(ix) S87N;
(x) S101M+ G118V+ S128L+P129Q+S130A;
(xi) S101G+S103A+V104I+Q245R;
(xii) S101R+S103A+V104I+G159S;
(xiii) S101G + V104N;
(xiv) S103A + V104I+G159D;
(xv) S103A + V104I;
(xvi) V68A+S101G+V104N; and
(xvii) Y167A + R170S + A194P

7. A composition according to any of the preceding claims wherein the protease further comprises additional amino acid substitutions versus SEQ ID NO: 1:
(i) G195E + M222A;
(ii) N76D + S87R + G118R+ S128L+P129Q+S130A;
(iii) S3T+S103A+V104I+G159S+V205I;
(iv) S9R+A15T+V68A+N218D;
(v) S87N + G118V+S128L+P129Q+S130A;
(vi) S101M+ G118V+ S128L+P129Q+S130A;
(vii) S101G+S103A+V104I+Q245R;
(viii) S101R+S103A+V104I+G159S;
(ix) S103A + V104I+G159D; and
(x) V68A+S101G+V104N;

8. A composition according to any of the preceding claims wherein the composition comprises less than 5%, preferably less than 2% by weight of the composition of anionic surfactant.

9. A composition according to any of the preceding claims wherein the composition has a pH of at least 9.5, preferably from 10.5 to 12 as measured in 1% by weight aqueous solution at 25°C.

10. A composition according to any of the preceding claims wherein composition comprises at least 10% by weight of the composition of a complexing agent.

11. A composition according to any of the preceding claims wherein the complexing agent is selected from the group consisting of citric acid, its salts and derivatives thereof, methyl glycine diacetic acid, its salts and derivatives thereof, glutamic-N,N- diacetic acid, its salts and derivatives thereof, iminodisuccinic acid, its salts and derivatives thereof, carboxy methyl inulin, its salts and derivatives thereof, and mixtures thereof, preferably the complexing agent is selected from the group consisting of methyl glycine diacetic acid, its salts and mixtures thereof.

12. A composition according to any of the preceding claims wherein the composition comprises a dispersant polymer, preferably a carboxylated/sulfonated polymer.

13. A composition according to any of the preceding claims wherein the composition comprises bleach.

14. A composition according to any of the preceding claims wherein the composition comprises a manganese bleach catalyst.

15. A composition according to any of the preceding claims wherein the composition comprises a crystal growth inhibitor.

16. A composition according to any of the preceding claims comprising an amylase.

17. A composition according to any of the preceding claims comprising an additional protease.

18. A composition according to any of the preceding claims comprising:
a) from 0.05 to 2 mg/g of the composition of the variant protease ;
b) from 0.025 to 0.3 mg/g of the composition of amylase;
c) from 5 to 20% by weight of the composition of bleach;
d) from 10 to 50% by weight of the composition of a complexing agent;
e) from 0.1 to 8% by weight of the composition of a crystal growth inhibitor; and
f) from 1 to 10% by weight of the composition of a dispersant polymer selected from the group consisting of carboxylated polymers free of sulfonated monomers, sulfonated polymers and mixtures thereof.

19. A composition according to any of the preceding claims wherein the composition is in unit dose form, preferably in the form of a water-soluble pack.

20. A method of automatic dishwashing comprising the following steps:
a) providing soiled dishware wherein the soil comprises burnt-on sugary food soils;
b) placing the dishware into an automatic dishwasher;
c) providing an automatic dishwashing cleaning composition according to any of claims 1 to 17; and
d) subjecting the dishware to an automatic dishwashing program.

21. A method of automatic dishwashing comprising the following steps:
a) providing soiled dishware;
b) placing the soiled dishware into an automatic dishwasher;
c) providing an automatic dishwashing cleaning composition according to any of claims 1 to 17; and
d) subjecting the dishware to a wash cycle at a temperature of 50°C or below.

22. A method of automatic dishwashing comprising the following steps:
a) providing soiled dishware;
b) placing the soiled dishware into an automatic dishwasher;
c) providing an automatic dishwashing cleaning composition according to any of claims 1 to 17; and
d) subjecting the dishware to a wash cycle to a wash cycle of equal or less than 30 mins.

23. Use of the composition according to any of claims 1 to 17 to clean crème brûlée in automatic dishwashing.


SEQUENCE LISTING

<110>  Procter & Gamble

<120>  AUTOMATIC DISHWASHING DETERGENT COMPOSITION

<130>  CM04545F

<160>  2

<170>  PatentIn version 3.5

<210>  1
<211>  269
<212>  PRT
<213>  Bacillus lentus GG36 subtilisin

<400>  1

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  2
<211>  270
<212>  PRT
<213>  Artificial Sequence

<220>
<223>  Variant of Bacillus lentus GG36 subtilisin

<400>  2

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Glu Gly Ser Gly Ser Val Ser Ser Ile Ala Gln Gly Leu Glu Trp
            100                 105                 110

Ala Gly Asn Asn Gly Met His Val Ala Asn Leu Ser Leu Gly Ser Pro

115                 120                 125

Ser Pro Ser Ala Thr Leu Glu Gln Ala Val Asn Ser Ala Thr Ser Arg
    130                 135                 140

Gly Val Leu Val Val Ala Ala Ser Gly Asn Ser Gly Ala Gly Ser Ile
145                 150                 155                 160

Ser Tyr Pro Ala Arg Tyr Ala Asn Ala Met Ala Val Gly Ala Thr Asp
                165                 170                 175

Gln Asn Asn Asn Arg Ala Ser Phe Ser Gln Tyr Gly Ala Gly Leu Asp
            180                 185                 190

Ile Val Ala Pro Gly Val Asn Val Gln Ser Thr Tyr Pro Gly Ser Thr
        195                 200                 205

Tyr Ala Ser Leu Asn Gly Thr Ser Met Ala Thr Pro His Val Ala Gly
    210                 215                 220

Ala Ala Ala Leu Val Lys Gln Lys Asn Pro Ser Trp Ser Asn Val Gln
225                 230                 235                 240

Ile Arg Asn His Leu Lys Asn Thr Ala Thr Ser Leu Gly Ser Thr Asn
                245                 250                 255

Leu Tyr Gly Ser Gly Leu Val Asn Ala Glu Ala Ala Thr Arg
            260                 265                 270